# EUROPÄISCHE PATENTSCHRIFT

(11) **EP 3 353 001 B1**
(45) Veröffentlichungstag und Bekanntmachung des Hinweises auf die Patenterteilung: **20.05.2020**
(21) Anmeldenummer: 16750848.0
(22) Anmeldetag: 12.08.2016
(51) Int. Cl.: B60H 1/00, G01N 33/00

(54) **INNENRAUMBELÜFTUNGSSYSTEM FÜR EIN KRAFTFAHRZEUG**
INTERIOR VENTILATION SYSTEM FOR A MOTOR VEHICLE
SYSTÈME DE VENTILATION DE L'HABITACLE D'UN VÉHICULE AUTOMOBILE

(30) Priorität: 25.09.2015 DE 102015218474
(43) Veröffentlichungstag der Anmeldung: 01.08.2018
(73) Patentinhaber: Bayerische Motoren Werke Aktiengesellschaft, 80809 München (DE)
(72) Erfinder: SEIFERLEIN, Mara, 80807 München (DE); BERNAU, Hendrik, 85221 Dachau (DE)
(86) Internationale Anmeldenummer: PCT/EP2016/069234
(87) Internationale Veröffentlichungsnummer: WO 2017/050485

(56) Entgegenhaltungen:
- DE-A1- 10 316 294
- DE-A1-102007 018 571
- DE-A1-102013 214 071

## Beschreibung

Die Erfindung betrifft ein Innenraumbelüftungssystem für Kraftfahrzeuge zur Messung einer Umgebungsluft und einer Innenraumluft des Fahrzeugs.

Aus der DE 102 54 496 A1 ist ein System und ein Verfahren zur Bekämpfung von Gerüchen und/oder Schadstoffen im Fahrzeuginnenraum bekannt. Das in dieser Druckschrift beschriebene System hat einige Sensoren zum Erfassen von Schadstoffen in einem Fahrzeuginnenraum sowie in einer Fahrzeugumgebung. Solche Innenraumbelüftungssysteme sind, auch aufgrund der Vielzahl an benötigten Sensoren, verhältnismäßig teuer.

Aus der DE 10 2013 214 071 A1 ist ferner ein Innenraumbelüftungssystem für ein Kraftfahrzeug bekannt. Dieses Innenraumbelüftungssystem weist eine Luftführungseinrichtung und in diese integrierte Luftgütesensoren auf. Diese messen je nach Betriebszustand der Luftführungseinrichtung mindestens einen Qualitätsparameter der im Bereich der Luftgütesensoren vorhandenen Umluft oder Frischluft oder der dort vorhandenen, Umluft und Frischluft enthaltenden Mischluft.

Ferner sind aus der DE 103 16 294 A1 und DE 10 2007 018 571 A1 Innenraumbelüftungssysteme bekannt, welche in diese integrierte Luftgütesensoren aufweisen.

Aufgabe der Erfindung ist es, ein Innenraumbelüftungssystem zur Erfassung von Schadstoffen bereitzustellen, welches eine kostengünstige und einfache Messung der Luftgüte ermöglicht. Diese Aufgabe wird mit einem Innenraumbelüftungssystem mit den Merkmalen des Patentanspruchs 1 gelöst. Vorteilhafte Weiterbildungen der Erfindung sind Gegenstand der abhängigen Ansprüche.

Gemäß einem Ausführungsbeispiel der Erfindung wird ein Innenraumbelüftungssystem für ein Kraftfahrzeug bereitgestellt, mit einem Luftgütesensorikmodul. Das Luftgütesensorikmodul hat einen Luftmessraum, in den über eine Außenluftzuleitung eine Umgebungsluft des Kraftfahrzeugs und über eine Innenluftzuleitung eine Innenraumluft des Kraftfahrzeugs zuführbar ist, Luftgütesensoren zur Messung eines Schadstoffgehalts in einer im Luftmessraum befindlichen Luft, und eine Messluftsteuereinrichtung, die angepasst ist, den Luftmessraum wahlweise mit Umgebungsluft und Innenraumluft zu versorgen. Darüber hinaus weist das Innenraumbelüftungssystem eine von dem Luftgütesensorikmodul separate Luftführungseinrichtung auf, die je nach Stellung eine Luft zur Innenraumbelüftung des Kraftfahrzeugs zumindest überwiegend aus einer Fahrzeugumgebung oder einem Fahrzeuginnenraum bezieht oder eine Innenraumbelüftung unterbricht. Ferner weist das Innenraumbelüftungssystem eine Steuereinrichtung auf, die die Luftführungseinrichtung abhängig von den Messergebnissen der Luftgütesensoren steuert. Dieses Ausführungsbeispiel bietet den Vorteil, dass zusammengefasste Sensoren, beispielsweise in Form eines kombinierten Luftgütesensorikmoduls, kostengünstiger sind als alle Luftgütesensoren einzeln zu kaufen. Ferner wird nur ein einziger Bauraum benötigt und somit nur ein einziger Masseanschluss, ein einziger Stromanschluss und eine einzige Kabelbaum- oder Busanbindung was den Aufwand für die Montage verringert. Das Ausführungsbeispiel ermöglicht, alle relevanten Luftgütewerte zentralisiert zu messen. Dabei bietet dieses Ausführungsbeispiel insbesondere gegenüber der DE 10 2013 214 071 A1 den Vorteil, dass die Luftgütemessung von Innenraumluft und Umgebungsluft unabhängig von der gerade durchgeführten Innenraumbelüftung erfolgen kann.

Gemäß einem weiteren Ausführungsbeispiel der Erfindung umfasst das Innenraumbelüftungssystem einen PM-Sensor zum Messen eines Feinstaub-Gehalts in der Umgebungsluft, einen NOₓ-Sensor zum Messen eines NOₓ-Gehalts in der Umgebungsluft, und einen CO-Sensor zum Messen eines CO-Gehalts in der Umgebungsluft. Damit sind die wichtigsten Luftgütewerte in der Umgebungsluft abgedeckt.

Gemäß einem weiteren Ausführungsbeispiel der Erfindung umfassen die Luftgütesensoren einen CO₂-Sensor zum Messen eines CO₂-Gehalts in der Innenraumluft, und die Steuereinrichtung ist angepasst, beim Überschreiten eines Grenzwertes des CO₂-Gehalts, die Luftführungseinrichtung so zu steuern, dass die Luft zur Innenraumbelüftung zumindest überwiegend aus der Fahrzeugumgebung bezogen wird. Dies hat den Vorteil, dass bei Klimaanlagen, welche CO₂ als Kühlmittel einsetzen, der CO₂-Sensor als sicherheitsrelevanter Sensor verwendet werden kann, der eine Leckage im Kühlmittelkreislauf detektieren kann. Ferner könnte ein zu hoher CO₂ Gehalt in der Innenraumluft zu Müdigkeit des Fahrers führen. Dem könnte durch Detektieren und Frischluftzufuhr vorgebeugt werden.

Gemäß einem weiteren Ausführungsbeispiel der Erfindung ist die Steuereinrichtung angepasst, beim Überschreiten zumindest eines Grenzwertes aus Feinstaub-Gehalt, NOₓ-Gehalt oder CO-Gehalt die Luftführungseinrichtung so zu steuern, dass die Luft zur Innenraumbelüftung zumindest überwiegend aus dem Fahrzeuginnenraum bezogen wird oder eine Luftzufuhr unterbunden wird, wenn nicht der Grenzwert des CO₂-Gehalts überschritten ist oder ein Scheibendetektor eine beschlagene Windschutzscheibe meldet. Somit werden bei der Innenraumbelüftung sicherheitsrelevante Aspekte priorisiert.

Gemäß einem weiteren Ausführungsbeispiel der Erfindung ist der Luftmessraum in einem Gehäuse ausgebildet, an dem die Luftgütesensoren befestigt sind, wobei das Gehäuse eine einzige Schnittstelle zur Anbindung an einen Fahrzeugbus aufweist. Dies verringert den Montage- und Verkabelungsaufwand.

Gemäß einem weiteren Ausführungsbeispiel schaltet die Messluftsteuereinrichtung, die Luftgütesensoren, welche zur Messung der Umgebungsluft vorgesehen sind, nur dann aktiv, wenn der Luftmessraum mit Umgebungsluft versorgt ist, und schaltet die Luftgütesensoren, welche zur Messung der Innenraumluft vorgesehen sind, nur dann aktiv, wenn der Luftmessraum mit Innenraumluft versorgt ist. Somit werden die Sensoren nur im Bedarfsfall aktiviert, was Energie sparen kann.

Gemäß einem weiteren Ausführungsbeispiel der Erfindung ist die Messluftsteuereinrichtung zur Steuerung von Luftströmungssteuerelementen angepasst, welche wahlweise Luft über die Außenluftzuleitung oder die Innenluftzuleitung fördern, wobei die Luftströmungssteuerelemente ein Gebläse, eine Pumpe und/oder Unterdruckerzeugungsmittel umfassen.

Gemäß einem weiteren Ausführungsbeispiel der Erfindung umfassen die Luftgütesensoren einen VOC-Sensor zum Messen eines Gehalts an flüchtigen Kohlenwasserstoffen in der Innenraumluft, und die Steuereinrichtung ist angepasst, beim Überschreiten eines Grenzwertes des VOC-Gehalts, die Luftführungseinrichtung so zu steuern, dass die Luft zur Innenraumbelüftung zumindest überwiegend aus der Fahrzeugumgebung bezogen wird.

Darüber hinaus stellt die Erfindung ein Kraftfahrzeug mit einem Innenraumbelüftungssystem gemäß einem der vorhergehenden Ausführungsbeispiele bereit.

Nachfolgend wird ein bevorzugtes Ausführungsbeispiel der vorliegenden Erfindung unter Bezugnahme auf die beigefügten Zeichnungen beschrieben. In diesen Zeichnungen ist Folgendes dargestellt:
- Figur 1: zeigt eine schematische Darstellung eines Luftgütesensorikmoduls der Erfindung, und
- Figur 2: zeigt schematisch ein Kraftfahrzeug mit einem Innenraumbelüftungssystem der Erfindung.

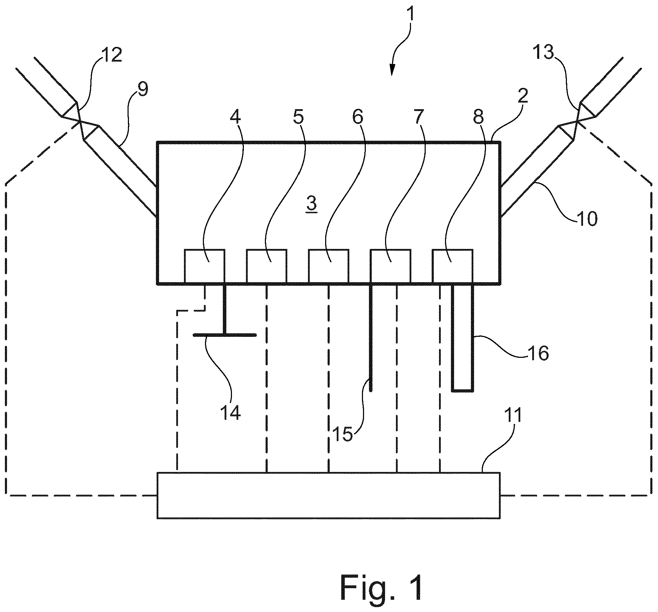

In Figur 1 ist schematisch ein Luftgütesensorikmodul 1 der Erfindung dargestellt. Dieses umfasst ein Gehäuse 2 in dem ein Luftmessraum 3 ausgebildet wird. An dem Gehäuse 2 sind mehrere Luftgütesensoren 4, 5, 6, 7 und 8 angeordnet und so mit dem Gehäuse verbunden, dass zumindest deren Messabschnitte in den Luftmessraum 3 ragen. Selbstverständlich können die sie auch vollständig im Gehäuse 2 angeordnet sein. Der Luftmessraum 3 ist abgesehen von einer Außenluftzuleitung 9, einer Innenluftzuleitung 10 und einem optionalen Auslass (nicht dargestellt) verschlossen. Über die Außenluftzuleitung 9 kann eine Umgebungsluft eines Kraftfahrzeugs 17, in dem das Luftgütesensorikmodul 1 montiert ist, in den Luftmessraum 3 eingeleitet werden. Über die Innenluftzuleitung 10 kann eine Innenraumluft des Kraftfahrzeugs 17 in den Luftmessraum 3 eingeleitet werden. Ein wesentlicher Aspekt der Erfindung ist, dass nicht wie beim Stand der Technik die Luftgütesensoren entfernt voneinander im Fahrzeug verteilt sind, sondern zusammengefasst sind und alle zur Schadstofferfassung innerhalb des Innenraumbelüftungssystems vorgesehenen Luftgütesensoren 4 bis 8 die Luft im selben Luftmessraum 3 messen. Dadurch können alle Luftgütesensoren 4 bis 8 in einem Gehäuse 2 zusammengefasst werden, was wiederum nur eine einzige Schnittstelle zur Anbindung an eine Steuereinrichtung des Fahrzeugs erfordert und eine Montage im Kraftfahrzeug 17 vereinfacht.

Darüber hinaus ist eine Messluftsteuereinrichtung 11 vorgesehen, diese kann elektrische Schaltkreise und Software umfassen. Die Messluftsteuereinrichtung 11 ist angepasst Luftströmungssteuerelemente 12, 13 zu steuern, diese sind vereinfacht in Form von Ventilen dargestellt. Diese sollen jedoch allgemein Mittel umfassen, die angepasst sind, die Luftströmungen durch die Außenluftzuleitung 9 und die Innenluftzuleitung 10 zu fördern und/oder zu unterbinden. So können die Luftströmungssteuerelemente 12, 13 beispielsweise Pumpen, Gebläse, Ventile, Klappen, Unterdruckeinrichtungen und Überdruckeinrichtungen umfassen. Die Funktion der Luftströmungssteuerelemente 12, 13 ist derart, dass diese eine Umgebungsluft in den Luftmessraum 3 fördern bzw. den Luftmessraum 3 mit Umgebungsluft füllen, wenn Schadstoffe in der Umgebungsluft gemessen werden sollen und eine Innenraumluft in den Luftmessraum 3 fördern bzw. den Luftmessraum 3 mit Innenraumluft füllen, wenn Schadstoffe in der Innenraumluft gemessen werden sollen. Die Luft, welche sich zuvor in dem Luftmessraum 3 befand, kann entweder über einen optionalen Auslass abgelassen werden oder sie wird über die jeweils nicht zur Einbringung von Luft benützte Zuleitung 9, 10 abgelassen oder hinausgepresst. Die Umschaltung zwischen Umgebungsluftmessung und Innenraumluftmessung wird von der Messluftsteuereinrichtung angesteuert und kann abwechselnd erfolgen oder von der Messluftsteuereinrichtung 11 gewählt werden.

Bei den Luftgütesensoren 4 bis 8 handelt es sich vorzugsweise um einen PM-Sensor 4 zum Messen eines Feinstaub-Gehalts in der Umgebungsluft, einen NOₓ-Sensor 5 zum Messen eines NOₓ-Gehalts in der Umgebungsluft, einen CO-Sensor 6 zum Messen eines CO-Gehalts in der Umgebungsluft, einen CO₂-Sensor 7 zum Messen eines CO₂-Gehalts in der Innenraumluft, und einem VOC-Sensor 8 zum Messen eines Gehalts an flüchtigen Kohlenwasserstoffen in der Innenraumluft. Der PM-Sensor 4 ist vorzugsweise angepasst, einen Feinstaub-Gehalt der Partikelgröße PM2.5 zu messen, er kann jedoch ebenso für jede andere Partikelgröße angepasst sein. Vorzugsweise ist dieser PM-Sensor 4 ein optischer Sensor. Die übrigen Luftgütesensoren 5 bis 8 sind vorzugsweise jeweils mit einem Mess-Chip versehen, auf den auf das Messmedium angepasste Pasten aufgebracht sind. Die Funktionsweisen der einzelnen Luftgütesensoren 4 bis 8 sind aus dem Stand der Technik bekannt und daher nicht weiter erläutert.

Das Luftsensorikmodul 1 umfasst des Weiteren einen Masseanschluss 14 und einen Stromanschluss 15, der beispielsweise ein Potential von +12V oder +5V aufweist. Ferner ist eine Schnittstelle 16 zum Verbinden des Luftsensorikmoduls 1 mit einem Fahrzeugbus vorgesehen. Der Vorteil ist, dass durch die Zusammenfassung der Luftgütesensoren nur eine einzige Schnittstelle 16 zur Anbindung aller Luftgütesensoren 4 bis 8 erforderlich ist. Dies spart Schnittstellen, Verkabelungs- und Einbauaufwand.

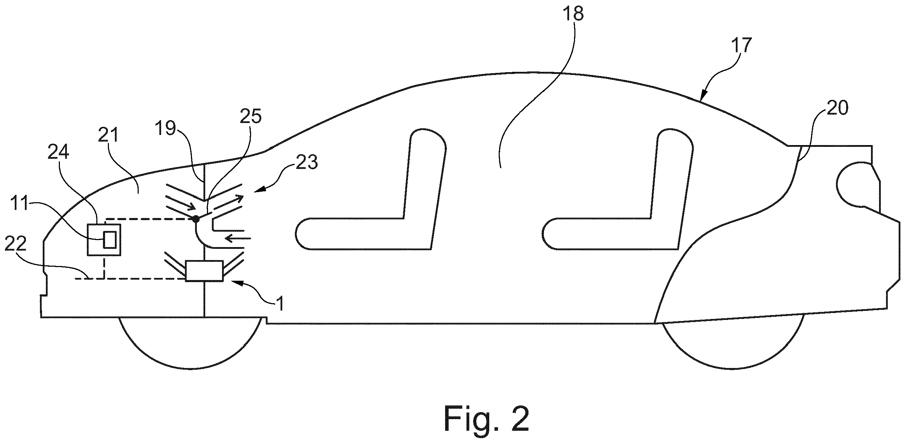

Figur 2 zeigt schematisch ein Kraftfahrzeug 17 mit einem Fahrzeuginnenraum 18, der insbesondere im Falle eines Autos vorn von einer Stirnwand 19 (auch als Spritzwand bezeichnet) begrenzt wird. Im Heck erstreckt sich der Fahrzeuginnenraum 18 entweder bis zu einem Kofferraum des Kraftfahrzeugs 17 oder wird durch eine Trennwand 20 vom Kofferraum abgegrenzt. Die Luft im Fahrzeuginnenraum 18 wird in dieser Beschreibung als Innenraumluft bezeichnet. Die das Fahrzeug 17 umgebende Luft ist die Umgebungsluft, die in der Regel auch in einem Motorraum 21 vorliegt.

Das Luftsensorikmodul 1 kann in der Stirnwand 19 oder in dessen Umgebung montiert sein. Über die Schnittstelle 16 ist das Luftsensorikmodul 1 mit einem Fahrzeugbus 22 (z.B. CAN, LIN, PWM) elektrisch verbunden, bei dem es sich bekanntermaßen um eine Datenleitung mit parallelen Leitungen handelt, wobei die Schnittstelle 16 mit allen oder zumindest mehreren der parallelen Leitungen elektrisch verbunden ist oder verbunden werden kann. Zur Kommunikation des Luftsensorikmoduls 1 mit anderen Steuerungselementen über den Fahrzeugbus 1 ist dem Luftsensorikmodul 1 eine eindeutige Kennung zugeordnet, die über den Fahrzeugbus 22 übertragen wird. Die Schnittstelle 16 kann auch als drahtlose Schnittstelle ausgeführt sein und mittels elektromagnetischer Wellen Daten austauschen, beispielsweise über Bluetooth, WLAN, NFC, usw.

Für die Belüftung des Fahrzeuginnenraums 18 ist eine Luftführungseinrichtung 23 vorgesehen, die stark schematisiert dargestellt ist. Funktion der Luftführungseinrichtung 23 ist es, je nach Stellung eine Luft zur Innenraumbelüftung des Kraftfahrzeugs 17 zumindest überwiegend (vorzugsweise im Wesentlichen vollständig) aus der Umgebung des Kraftfahrzeugs 17 oder dem Fahrzeuginnenraum 18 des Kraftfahrzeugs 17 zu beziehen oder eine Innenraumbelüftung zu unterbrechen. Die Luftführungseinrichtung 23 wird von einer Steuereinrichtung 24, die elektrische Schaltkreise und Software umfassen kann, in Abhängigkeit von den Messergebnissen der Luftgütesensoren 4 - 8 angesteuert, die sie von diesen direkt oder über die Messluftsteuereinrichtung 11 erhält. Die Steuereinrichtung 24 kann die Messluftsteuereinrichtung 11 enthalten, die Messluftsteuereinrichtung 11 kann im Luftsensorikmodul 1 ausgebildet sein oder die Messluftsteuereinrichtung 11 kann separat bereitgestellt werden. Die Steuereinrichtung 24 ist ebenfalls mit dem Fahrzeugbus 22 verbunden. Im Betrieb des Innenraumbelüftungssystems wird neben den Wünschen des Fahrgastes, der manuell eine Luftzufuhr zur Innenraumbelüftung zwischen Umgebungsluftzufuhr und Innenraumluftumwälzung wählen kann, ein Automatikbetrieb bereitgestellt, der die Luftführungseinrichtung 23 in Abhängigkeit von den Messergebnissen der Luftgütesensoren so steuert, dass die Luft bei der Wahl zwischen Umgebungsluft und Innenraumluft von dort bezogen wird, wo sie für den menschlichen Organismus weniger schädlich ist. Innerhalb der Luftführungseinrichtung 23 ist zum Zwecke dieser Luftführung beispielsweise eine Umluftklappe 25 vorgesehen. Hierzu ist die Steuereinrichtung 24 angepasst, die Luftführungseinrichtung so zu steuern, dass bei Überschreiten eines vorbestimmten Grenzwertes eines die Umgebungsluft betreffenden Grenzwertes, insbesondere des NOₓ-Gehalts, des CO-Gehalts und/oder des Feinstaub-Gehalts, die Luft zur Innenraumbelüftung des Kraftfahrzeugs zumindest überwiegend (vorzugsweise im Wesentlichen vollständig) aus dem Fahrzeuginnenraum 18 des Kraftfahrzeugs 17 bezogen wird oder eine Innenraumbelüftung unterbrochen wird. Ferner ist die Steuereinrichtung 24 angepasst, die Luftführungseinrichtung so zu steuern, dass bei Überschreiten eines vorbestimmten Grenzwertes eines die Innenraumluft betreffenden Grenzwertes, insbesondere des VOC-Gehalts und/oder des CO₂-Gehalts, die Luft zur Innenraumbelüftung des Kraftfahrzeugs zumindest überwiegend (vorzugsweise im Wesentlichen vollständig) aus der Umgebung des Kraftfahrzeugs 17 bezogen wird. Sollten vorbestimmte Grenzwerte sowohl in der Umgebungsluft als auch in der Innenraumluft überschritten werden, dann führt die Steuereinrichtung 24 eine Gewichtung durch, welche Schadstoffe schädlicher sind oder zwingend eine bestimmte Luftzufuhr erfordern. Ist beispielsweise ein bestimmter Grenzwert des CO₂-Gehalts der Innenraumluft überschritten, dann wird zwangsläufig die Luft zur Innenraumbelüftung des Kraftfahrzeugs aus der Umgebung des Kraftfahrzeugs 17 bezogen. Ebenfalls wird beim Beschlagen der Windschutzscheibe, was beispielsweise von einem Scheibendetektor erfasst werden kann, zwangsläufig die Luft zur Innenraumbelüftung des Kraftfahrzeugs aus der Umgebung des Kraftfahrzeugs 17 bezogen.

Zur Information der Fahrzeuginsassen, kann eine visuelle oder akustische Information und/oder Warnmeldung ausgegeben werden, die über die ermittelte Luftgüte informiert. Beispielsweise kann das Überschreiten bestimmter Luftgüte-Grenzwerte angezeigt werden und die Fahrzeuginsassen können über Maßnahmen informiert werden, welche das Fahrzeug unternimmt und/oder die Fahrzeuginsassen zu unternehmen haben.

Darüber hinaus kann das erfindungsgemäße Innenraumbelüftungssystem eine aktive Luftreinigungsvorrichtung aufweisen, welche die Luft durch Filtrierung und/oder Ionisation reinigt. Hierzu wird die in den Fahrzeuginnenraum einzuführende Luft durch die Luftreinigungsvorrichtung gefördert.

Während die Erfindung detailliert in den Zeichnungen und der vorangehenden Beschreibung veranschaulicht und beschrieben wurde, ist diese Veranschaulichung und Beschreibung als veranschaulichend oder beispielhaft und nicht als beschränkend zu verstehen und es ist nicht beabsichtigt die Erfindung auf die offenbarten Ausführungsbeispiele zu beschränken.

### Bezugszeichenliste:

- 1: Luftgütesensorikmodul
- 2: Gehäuse
- 3: Luftmessraum
- 4: PM-Sensor (Luftgütesensor)
- 5: NOₓ-Sensor (Luftgütesensor)
- 6: CO-Sensor (Luftgütesensor)
- 7: CO₂-Sensor (Luftgütesensor)
- 8: VOC-Sensor (Luftgütesensor)
- 9: Außenluftzuleitung
- 10: Innenluftzuleitung
- 11: Messluftsteuereinrichtung
- 12: Luftströmungssteuerelement
- 13: Luftströmungssteuerelement
- 14: Masseanschluss
- 15: Stromanschluss
- 16: Schnittstelle
- 17: Kraftfahrzeug
- 18: Fahrzeuginnenraum
- 19: Stirnwand
- 20: Trennwand
- 21: Motorraum
- 22: Fahrzeugbus
- 23: Luftführungseinrichtung
- 24: Steuereinrichtung
- 25: Umluftklappe

## Patentansprüche

1. Innenraumbelüftungssystem für ein Kraftfahrzeug (17), mit
einem Luftgütesensorikmodul (1), mit
einen Luftmessraum (3), in den über eine Außenluftzuleitung (9) eine Umgebungsluft des Kraftfahrzeugs (17) und über eine Innenluftzuleitung (10) eine Innenraumluft des Kraftfahrzeugs (17) zuführbar ist,
Luftgütesensoren (4 - 8) zur Messung eines Schadstoffgehalts in einer im Luftmessraum (3) befindlichen Luft, und
eine Messluftsteuereinrichtung (11), die angepasst ist, den Luftmessraum (3) wahlweise mit Umgebungsluft und Innenraumluft zu versorgen;
einer von dem Luftgütesensorikmodul (1) separaten Luftführungseinrichtung (23), die je nach Stellung eine Luft zur Innenraumbelüftung des Kraftfahrzeugs (17) zumindest überwiegend aus einer Fahrzeugumgebung oder einem Fahrzeuginnenraum (18) bezieht oder eine Innenraumbelüftung unterbricht, und
einer Steuereinrichtung (24), die die Luftführungseinrichtung (23) abhängig von den Messergebnissen der Luftgütesensoren (4 - 8) steuert.

2. Innenraumbelüftungssystem gemäß Anspruch 1, wobei die Luftgütesensoren (4 - 8)
einen PM-Sensor (4) zum Messen eines Feinstaub-Gehalts in der Umgebungsluft,
einen NOₓ-Sensor (5) zum Messen eines NOₓ-Gehalts in der Umgebungsluft, und
einen CO-Sensor (6) zum Messen eines CO-Gehalts in der Umgebungsluft umfassen.

3. Innenraumbelüftungssystem gemäß einem der vorhergehenden Ansprüche, wobei die Luftgütesensoren (4 - 8)
einen CO₂-Sensor (7) zum Messen eines CO₂-Gehalts in der Innenraumluft umfassen, und die Steuereinrichtung (24) angepasst ist, beim Überschreiten eines Grenzwertes des CO₂-Gehalts, die Luftführungseinrichtung (23) so zu steuern, dass die Luft zur Innenraumbelüftung zumindest überwiegend aus der Fahrzeugumgebung bezogen wird.

4. Innenraumbelüftungssystem gemäß Anspruch 3, wobei die Steuereinrichtung (24) angepasst ist, beim Überschreiten zumindest eines Grenzwertes aus Feinstaub-Gehalt, NOₓ-Gehalt oder CO-Gehalt die Luftführungseinrichtung (23) so zu steuern, dass die Luft zur Innenraumbelüftung zumindest überwiegend aus dem Fahrzeuginnenraum (18) bezogen wird oder eine Luftzufuhr unterbunden wird, wenn nicht der Grenzwert des CO₂-Gehalts überschritten ist oder ein Scheibendetektor eine beschlagene Windschutzscheibe meldet.

5. Innenraumbelüftungssystem gemäß einem der vorhergehenden Ansprüche, wobei der Luftmessraum (3) in einem Gehäuse (2) ausgebildet ist, an dem die Luftgütesensoren (4 - 8) befestigt sind, wobei das Gehäuse (2) eine einzige Schnittstelle (16) zur Anbindung an einen Fahrzeugbus (22) aufweist.

6. Innenraumbelüftungssystem gemäß einem der vorhergehenden Ansprüche, wobei die Messluftsteuereinrichtung (11), die Luftgütesensoren (4, 5, 6), welche zur Messung der Umgebungsluft vorgesehen sind, nur dann aktiv schaltet, wenn der Luftmessraum (3) mit Umgebungsluft versorgt ist, und die Luftgütesensoren (7, 8), welche zur Messung der Innenraumluft vorgesehen sind, nur dann aktiv schaltet, wenn der Luftmessraum mit Innenraumluft versorgt ist.

7. Innenraumbelüftungssystem gemäß einem der vorhergehenden Ansprüche, wobei die Messluftsteuereinrichtung (11) zur Steuerung von Luftströmungssteuerelementen (12, 13) angepasst ist, welche wahlweise Luft über die Außenluftzuleitung (9) oder die Innenluftzuleitung (10) fördern, wobei die Luftströmungssteuerelemente (12, 13) ein Gebläse, eine Pumpe und/oder Unterdruckerzeugungsmittel umfassen.

8. Innenraumbelüftungssystem gemäß einem der vorhergehenden Ansprüche, wobei die Luftgütesensoren (4 - 8) einen VOC-Sensor (8) zum Messen eines Gehalts an flüchtigen Kohlenwasserstoffen in der Innenraumluft umfassen, und die Steuereinrichtung (24) angepasst ist, beim Überschreiten eines Grenzwertes des VOC-Gehalts, die Luftführungseinrichtung (23) so zu steuern, dass die Luft zur Innenraumbelüftung zumindest überwiegend aus der Fahrzeugumgebung bezogen wird.

9. Kraftfahrzeug (17) mit einem Innenraumbelüftungssystem gemäß einem der vorhergehenden Ansprüche.

## Claims

1. An interior ventilation system for a motor vehicle (17), with
an air quality sensor system module (1), with
an air measurement chamber (3), into which ambient air of the motor vehicle (17) can be fed by way of an external-air feed line (9) and interior air of the motor vehicle (17) can be fed by way of an internal-air feed line (10),
air quality sensors (4 - 8) for measuring a pollutant content in air located in the air measurement chamber (3), and
a measurement-air control means (11), which is adapted to supply the air measurement chamber (3) selectively with ambient air and interior air;
an air guidance means (23) separate from the air quality sensor system module (1), which depending on its setting draws air for interior ventilation of the motor vehicle (17) at least predominantly from the vehicle surroundings or from a vehicle interior (18), or interrupts interior ventilation, and
a control means (24) which controls the air guidance means (23) depending on the measurement results of the air quality sensors (4 - 8).

2. An interior ventilation system according to Claim 1, wherein the air quality sensors (4 - 8)
comprise a PM sensor (4) for measuring a particulate content in the ambient air,
an NOₓ sensor (5) for measuring an NOₓ content in the ambient air, and
a CO sensor (6) for measuring a CO content in the ambient air.

3. An interior ventilation system according to one of the preceding claims, wherein the air quality sensors (4 - 8)
comprise a CO₂ sensor (7) for measuring a CO₂ content in the interior air, and the control means (24) is adapted, upon a limit value of the CO₂ content being exceeded, to control the air guidance means (23) such that the air for interior ventilation is drawn at least predominantly from the vehicle surroundings.

4. An interior ventilation system according to Claim 3, wherein the control means (24) is adapted, upon at least one limit value from particulate content, NOₓ content or CO content being exceeded, to control the air guidance means (23) such that the air for interior ventilation is drawn at least predominantly from the vehicle interior (18) or a supply of air is prevented if the limit value for the CO₂ content is not exceeded or a windscreen detector does not report a fogged windscreen.

5. An interior ventilation system according to one of the preceding claims, wherein the air measurement chamber (3) is formed in a housing (2) to which the air quality sensors (4 - 8) are fastened, wherein the housing (2) has a single interface (16) for attaching to a vehicle bus (22).

6. An interior ventilation system according to one of the preceding claims, wherein the measurement-air control means (11) switches the air quality sensors (4, 5, 6) which are provided for measuring the ambient air to active only if the air measurement chamber (3) is supplied with ambient air, and switches the air quality sensors (7, 8) which are provided for measuring the interior air to active only if the air measurement chamber is supplied with interior air.

7. An interior ventilation system according to one of the preceding claims, wherein the measurement-air control means (11) is adapted for controlling airflow control elements (12, 13) which selectively convey air by way of the external-air feed line (9) or the internal-air feed line (10), wherein the airflow control elements (12, 13) comprise a blower, a pump and/or vacuum generation means.

8. An interior ventilation system according to one of the preceding claims, wherein the air quality sensors (4 - 8) comprise a VOC sensor (8) for measuring a content of volatile hydrocarbons in the interior air, and the control means (24) is adapted, upon a limit value of the VOC content being exceeded, to control the air guidance means (23) such that the air for interior ventilation is drawn at least predominantly from the vehicle surroundings.

9. A motor vehicle (17) with an interior ventilation system according to one of the preceding claims.

## Revendications

1. Système de ventilation de l'habitacle d'un véhicule automobile (17) équipé d'un module de capteurs de la qualité de l'air (1) comprenant :
un espace de mesure de l'air (3) recevant l'air de l'habitacle du véhicule (17), par une conduite d'air extérieur (9), une conduite d'air ambiant du véhicule (17) et une conduite d'air intérieur (10),
des capteurs de qualité de l'air (4-8) pour mesurer la teneur en polluants dans l'air de l'espace de mesure d'air (3), et
une installation de commande de l'air de mesure (11) adapté pour alimenter l'espace de mesure d'air (3) sélectivement avec de l'air ambiant et de l'air de l'habitacle,
une installation de conduite d'air (23) distincte du module de capteurs de qualité d'air (1), et qui, selon la position, prélève de l'air pour la ventilation de l'habitacle du véhicule (17) au moins en grande partie de l'environnement du véhicule ou de l'habitacle (18) du véhicule ou encore coupe la ventilation de l'habitacle, et
une installation de commande (24) qui commande l'installation de conduite d'air (23) en fonction des résultats des mesures des capteurs de qualité de l'air (4-8).

2. Système de ventilation de l'habitacle selon la revendication 1,
dans lequel les capteurs de qualité d'air (4-8) comprennent :
un capteur PM (4) pour mesurer la teneur en particules fines de l'air ambiant,
un capteur NOₓ (5) pour mesurer la teneur en oxydes d'azote NOₓ de l'air ambiant, et
un capteur CO (6) pour mesurer la teneur en oxydes de carbone dans l'air ambiant.

3. Système de ventilation de l'habitacle selon l'une des revendications précédentes, dans lequel les capteurs de la qualité de l'air (4-8) commandent :
un capteur CO₂ (7) pour mesurer la teneur en CO₂ dans l'air de l'habitacle et l'installation de commande (24) est adaptée pour qu'en cas de dépassement d'un seuil de teneur en CO₂, commander l'installation de ventilation (23) pour que l'air servant à la ventilation de l'habitacle provienne au moins en grande partie de l'environnement du véhicule.

4. Système de ventilation de l'habitacle selon la revendication 3,
selon lequel l'installation de commande (24) est adaptée pour qu'en cas de dépassement d'au moins un seuil de teneur en particules fines, de la teneur en NOₓ ou de la teneur en CO, commander l'installation de guidage d'air (23) pour que l'air servant à la ventilation de l'habitacle provienne au moins en grande partie de l'habitacle (18) ou encore pour couper l'alimentation en air si le seuil de la teneur en CO₂ n'est pas dépassé ou si un détecteur de vitre signale que le pare-brise est embué.

5. Système de ventilation de l'habitacle selon l'une des revendications précédentes,
selon lequel l'espace de mesure d'air (3) est réalisé dans un boîtier (2) dans lequel sont fixés les capteurs de qualité d'air (4-8), le boîtier (2) ayant une unique interface (16) pour être relié au bus (22) du véhicule.

6. Système de ventilation de l'habitacle selon l'une des revendications précédentes,
selon lequel l'installation de commande de l'air de mesure (11) ne commute à l'état actif, les capteurs de qualité d'air (4, 5, 6) servant à mesurer l'air ambiant que si l'espace de mesure d'air (3) est alimenté en air ambiant et les capteurs de la qualité de l'air (7, 8) prévus pour mesurer l'air de l'habitacle ne sont activés que si l'espace de mesure est alimenté en air de l'habitacle.

7. Système de ventilation de l'habitacle selon l'une des revendications précédentes,
selon lequel l'installation de commande de l'air de mesure (11) est adapté pour commander les éléments de commande de circulation d'air (12, 13) qui fournissent sélectivement de l'air par la conduite d'alimentation en air extérieur (9) ou la conduite d'alimentation en air intérieur (10), les éléments de commande de la circulation d'air (12, 13) comprennent une soufflante, une pompe et/ou un moyen générant une dépression.

8. Système de ventilation de l'habitacle selon l'une des revendications précédentes,
selon lequel les capteurs de la qualité de l'air (4-8) comprennent un capteur VOC (composants organiques volatiles) (8) pour mesurer la teneur en hydrocarbures volatiles dans l'air de l'habitacle et l'installation de commande (24) est adaptée pour qu'en cas de dépassement d'un seuil de teneur VOC, l'installation de guidage de l'air (23) soit commandée pour que l'air pour la ventilation de l'habitacle soit prélevé au moins en grande partie de l'environnement du véhicule.

9. Véhicule (17) équipé d'un système d'aération de l'habitacle selon l'une des revendications précédentes.
